# EUROPÄISCHE PATENTSCHRIFT

(11) **EP 1 265 551 B1**
(45) Veröffentlichungstag und Bekanntmachung des Hinweises auf die Patenterteilung: **14.02.2007**
(21) Anmeldenummer: 01919224.4
(22) Anmeldetag: 20.03.2001
(51) Int. Cl.: A61F 2/00, A61F 2/02

(54) **IMPLANTIERBARE SPHINKTERPROTHESE**
IMPLANTABLE SPHINCTER PROSTHESIS
PROTHESE DE SPHINCTER POUVANT ETRE IMPLANTEE

(30) Priorität: 20.03.2000 DE 10013519
(43) Veröffentlichungstag der Anmeldung: 18.12.2002
(73) Patentinhaber: Adeva Medical Gesellschaft für Entwicklung und Vertrieb von Medizinischen Implantat-Artikeln mbH, 23556 Lübeck (DE)
(72) Erfinder: GRUNDEI, Hans, 23558 Lübeck (DE); MÜNDER, Ulrich, 23556 Lübeck (DE)
(74) Vertreter: Vollmann, Heiko
(86) Internationale Anmeldenummer: PCT/DE2001/001065
(87) Internationale Veröffentlichungsnummer: WO 2001/070131

(56) Entgegenhaltungen:
- EP-A- 0 421 557
- DE-A- 19 845 292
- FR-A- 2 756 485
- GB-A- 2 261 169
- US-A- 4 721 509

## Beschreibung

Die Erfindung betrifft eine implantierbare Sphinkterprothese zur Anwendung bei Harninkontinenz gemäß den im Oberbegriff des Anspruchs 1 angegebenen Merkmalen.

Eine derartige implantierbare Sphinkterprothese ist beispielsweise aus der DE-A-19 845 292 oder der FR-A-2 756 485 bekannt. Die aus der DE 195 11 998 A1 bekannte Sphinkterprothese besteht im Wesentlichen aus einer um die Harnröhre legbaren Manschette, an der ein Blähkörper so angeordnet ist, dass dieser durch Druckbeaufschlagung mittels einer Flüssigkeit dilatiert und dadurch die Harnröhre absperrt. Dieser Blähkörper ist über eine Schlauchleitung mit dem saugseitigen Anschluss einer Pumpe verbunden, deren druckseitiger Anschluss mit einem Flüssigkeitsreservoir leitungsverbunden ist, das innerhalb der Bauchhöhle liegt und von dem dort herrschenden Druck mechanisch beaufschlagt ist. Die Pumpe, welche auch ein Rückschlagventil beinhaltet, das deaktivierbar ist, wird im Scrotum oder Labium implantiert, so dass sowohl Pumpe als auch Ventil von außen betätigbar sind. Zum urinieren wird die Pumpe betätigt, so dass Flüssigkeit vom Blähkörper in das Reservoir gepumpt wird, ein Rückfluss wird durch das Rückschlagventil verhindert. Durch das Abpumpen der Flüssigkeit aus dem Blähkörper schrumpft dieser zusammen, wodurch auch der Druck auf die Harnröhre nachlässt und der Urinfluss durch dieselbe freigegeben wird. Nach Entleeren der Blase wird das Rückschlagventil deaktiviert, so dass die Flüssigkeit von dem im Bauchraum befindlichen Reservoir aufgrund des dort herrschenden Druckes wieder in den Blähkörper gelangt und schließlich die Harnröhre durch erneute Druckbeaufschlagung absperrt. Die Handhabung dieser Sphinkterprothese erfolgt ausschließlich manuell und kann Probleme (z. B. bei Handhabung durch Pflegepersonal) bereiten, insbesondere bei älteren Leuten, und zwar sowohl hinsichtlich der Erreichbarkeit als auch hinsichtlich der Handhabung selbst.

Vor diesem Stand der Technik liegt der Erfindung die Aufgabe zugrunde, eine gattungsgemäße Sphinkterprothese so weiterzubilden, dass die Handhabung für den Patienten vereinfacht wird.

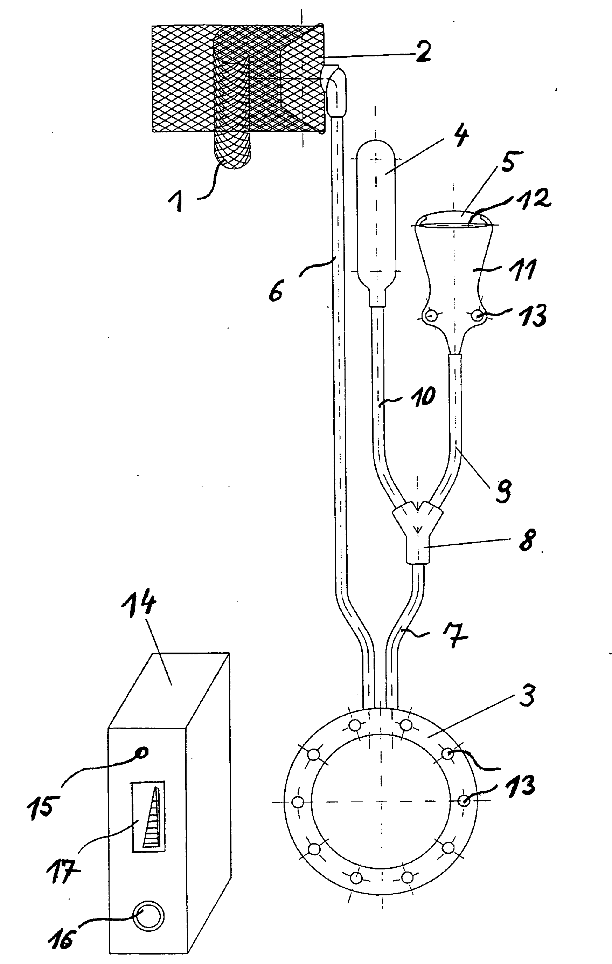

Diese Aufgabe wird gemäß der Erfindung durch die in Anspruch 1 angegebenen Merkmale gelöst. Vorteilhafte Ausgestaltungen der Erfindung sind in den Unteransprüchen, der nachfolgenden Beschreibung sowie der Zeichnung angegeben.

Grundgedanke der vorliegenden Erfindung ist es, anstelle einer manuell betätigbaren mechanischen Pumpe und eines manuell steuerbaren Ventils eine elektrisch angetriebene miniaturisierte Pumpe innerhalb des Körpers einzusetzen und diese über eine extrakorporale Steuer- und Versorgungseinrichtung drahtlos mit Energie zu versorgen und zu steuern. Wenn, was zweckmäßig ist, die extrakorporale Steuer- und Versorgungseinrichtung in Form eines mobilen Gerätes vorgesehen ist, kann der Patient die Pumpe dadurch betätigen, dass er dieses Gerät in eine entsprechend körpernahe Stellung bringt und zum Betätigen der Pumpe auslöst, wonach die Pumpe Fluid aus dem Blähkörper in das Reservoir pumpt, die Harnröhre in an sich bekannter Weise freigegeben wird, so dass die Blase entleert werden kann und nach Deaktivieren der Pumpe sich entweder der Blähkörper selbsttätig wieder füllt, wenn beispielsweise ein Überströmkanal (Bypass) vorgesehen ist, oder aber andere Mittel vorgesehen sind, um den Blähkörper wieder zu befüllen. Mit der erfindungsgemäßen Lösung kann der Patient sehr komfortabel und ohne eine bestimmte Körperstelle suchen und manuell beaufschlagen zu müssen, für seine Blasenentleerung sorgen. Da die Energieversorgung drahtlos von außen erfolgt, kann die Prothese vollständig implantiert werden, ohne dass irgendein Zugang durch die Haut erforderlich wäre. Dabei erfolgt die Energieversorgung zweckmäßigerweise induktiv mittels gepulstem Gleichstrom was sowohl günstig ist hinsichtlich des Aufbaus des extrakorporalen Steuer- und Versorgungsgeräts als auch in Hinblick auf die Übertragung und Umwandlung der in das Körperinnere übertragenen Energie. Es kann nämlich für die Energieversorgung innerhalb des Geräts eine Gleichstromquelle, vorzugsweise ein Akku oder auch eine Brennstoffzelle eingesetzt werden, wobei die Erzeugung eines gepulsten Gleichstroms mittels eines elektronischen Schalters, eines Kondensators und einer entsprechenden Steuerung in einfacher Weise erfolgen kann.

Zwar ist es grundsätzlich in einfachster Ausgestaltung möglich, den elektrischen Antrieb der Pumpe unmittelbar induktiv dadurch anzusteuern, dass dieser entsprechend induktiv versorgt wird, doch wird es schon aus Gründen der Betätigungssicherheit zweckmäßig sein, eine gesonderte Steuerung vorzusehen, die erst dann aktivierbar ist, wenn die Energieversorgung mit Signalübertragung zwischen dem extrakorporalen Gerät und einer die Pumpe steuernden intrakorporalen elektronischen Steuer- und Versorgungseinrichtung sichergestellt ist.

Es versteht sich, dass die intrakorporalen Bauteile, insbesondere die Pumpe eine möglichst kleine Baugröße haben sollten. In diesem Zusammenhang als besonders vorteilhaft erwiesen hat sich der Einsatz einer miniaturisierten Membranpumpe mit piezoelektrischem Antrieb. Eine solche Anordnung ist auch hinsichtlich der Ansteuerung und Energieumwandlung innerhalb der intrakorporalen Steuer- und Versorgungseinrichtung besonders günstig, da der intern durch Kondensator gepulste Gleichstrom zum Antrieb der Pumpe unmittelbar ohne weitere Umwandlung dienen kann, um den oder die Piezokristalle entsprechend anzusteuern. Bei jeder Spannungsbeaufschlagung dehnt sich der Piezokristall aus, wodurch die damit verbundene Membran der Pumpe ausgelenkt wird. Alternativ können auch elektromotorisch angetriebene Pumpen, beispielsweise Zahnrad- oder Kreiselpumpen eingesetzt werden, wobei dann in der intrakorporalen elektronischen Steuer- und Versorgungseinrichtung eine entsprechende Signalaufbereitung z. B. mittels eines Frequenzumrichters zur Ansteuerung eines Asynchronmotors oder anderer geeigneter Weise erfolgt. Dann kann es zweckmäßig sein, die induktive Übertragung mit Wechselstrom vorzusehen.

Um die Anzahl der innerhalb des Körpers zu fixierenden Bauteile möglichst gering zu halten, ist es von Vorteil, die Pumpe in eine Pumpeneinheit (als Baueinheit) zu integrieren, welche neben der Pumpe und deren elektrischen Antrieb auch die intrakorporale elektronische Steuer- und Versorgungseinrichtung sowie das Umschaltventil beinhaltet.

Bevorzugt wird im Bereich der Manschette ein Drucksensor vorgesehen, der so angeordnet und ausgelegt ist, dass der vom Blähkörper auf die Harnröhre wirkende Druck bzw. die dort wirkende Kraft ermittelt wird. Ein solcher Drucksensor ist zum einen zum Kalibrieren des Systems, das heißt zum Einstellen des Schließdrucks sehr nützlich, kann jedoch zum anderen auch zum Ansteuern der Pumpe genutzt werden. Zum Kalibrieren des Systems ist zudem ein intrakorporaler Port zweckmäßigerweise vorzusehen, der mit dem Reservoir und oder dem Blähkörper leitungsverbunden ist. Ein solcher Port wird so innerhalb des subkutanen Gewebes fixiert, dass von außen, beispielsweise durch Einstechen mittels einer Kanüle zunächst durch die Haut und dann in den Port Fluid zu- oder abgeführt werden kann, je nachdem, ob der Druck erhöht oder gesenkt werden soll.

Der manschettenseitige Drucksensor kann jedoch nicht nur zum Kalibrieren eingesetzt werden, sondern auch so, dass die Pumpe in Abhängigkeit des Sensorsignals gesteuert wird. Der Pumpe ist ein steuerbares Umschaltventil zugeordnet, das in Abhängigkeit des Sensorsignals gesteuert wird. Ein solches Umschaltventil kann als 2/2-Wege-Ventil ausgebildet sein und je nach Schaltstellung die Saug- und Druckanschlüsse vertauschen, so dass trotz gleichbleibender Förderrichtung in einer Schaltstellung Fluid von Blähkörper in das Reservoir und in der anderen Schaltstellung in umgekehrter Richtung gepumpt wird.

Besonders vorteilhaft ist es, wenn das gesamte zu implantierende System als zusammenhängendes fluidgefülltes und geschlossenes System eine Baueinheit bildet, so dass die bei bekannten Systemen vor oder während des Eingriffs vorzunehmenden Montagearbeiten und die damit verbundenen Risiken entfallen. Dabei wird der Blähkörper zusammen mit der um die Harnröhre legbaren Manschette über eine Schlauchleitung mit der Pumpeneinheit verbunden sein, die über eine weitere Schlauchleitung mit dem Reservoir verbunden ist, wobei an geeigneter Stelle noch der Port angeschlossen ist. Ein so gebildetes geschlossenes System ist wesentlich einfacher und günstiger zu handhaben als ein aus Einzelteilen zusammengesetztes und vom Anwender zu befüllendes und zu entlüftendes System.

Das die extrakorporale Steuer- und Versorgungseinrichtung beinhaltende Gerät kann klein und ergonomisch gestaltet werden. Zweckmäßigerweise wird das Gerät eine optische oder auch akustische Anzeigevorrichtung aufweisen, welche dem Benutzer anzeigt, dass das Gerät in der bestimmungsgemäßen Position an entsprechender Stelle auf der Haut des Patienten gebracht worden ist, in der die Energieversorgung der intrakorporalen Steuer- und Versorgungseinheit sichergestellt ist. Der Benutzer wird also erst dann, wenn er dieses Signal wahrgenommen hat, eine entsprechende Steuerfunktion, beispielsweise durch Betätigen eines Tasters auslösen, die dann die Pumpe in Betrieb setzt und gegebenenfalls den gesamten Vorgang zum Entleeren der Blase auch zeitmäßig steuert. Zweckmäßigerweise weist dieses Gerät auch eine Anzeige auf, welche den Ladezustand des Energiespeichers, beispielsweise Akkus, anzeigt.

Die Erfindung ist nachfolgend anhand eines in der Zeichnung dargestellten Ausführungsbeispiels näher erläutert. Die einzige Figur zeigt in stark vereinfachter schematischer Darstellung den Aufbau der erfindungsgemäßen Sphinkterprothese sowie das extrakorporale Steuer- und Versorgungsgerät.

Der implantierbare Teil der dargestellten Sphinkterprothese besteht im Wesentlichen aus vier Teilen, nämlich einer Manschette 1 mit daran angeordnetem Blähkörper 2, einer Pumpeneinheit 3, einem Reservoir 4 und einem Port 5. die über weiter unten noch beschriebene Schlauchleitungen miteinander verbunden sind.

Die Manschette 1 besteht aus einem Netzgewebe, in das ein dilatierbarer Blähkörper 2 aus elastischem Kunststoff eingearbeitet ist. Diese Manschette 1 wird um die Harnröhre, z. B. im Bereich des Blasenhalses gelegt, derart, dass beim Dilatieren des Blähkörpers 2 die Harnröhre zusammengedrückt, und damit ein Austreten von Ham aus der Blase durch die Harnröhre verhindert wird. Die Manschette 1 wird nach Umlegen vernäht, so dass sich ein geschlossener Manschettenring ergibt. Der Blähkörper 2 ist über eine aus Silikon bestehende Schlauchleitung 6 mit der Pumpeneinheit 3 verbunden. Diese Schlauchleitung 6 führt zu einem nicht dargestellten Sauganschluss einer Membranpumpe, die piezoelektrisch angetrieben ist. Der Druckanschluss der Pumpe ist über eine Schlauchleitung 7 mit einem Y-Stück 8 verbunden, welches die Schlauchleitung 7 in zwei Schlauchleitungen 9 und 10 aufteilt.

Die Schlauchleitung 9 ist mit dem Port 5 verbunden, der aus einem metallischen Gehäuse 11 besteht, dass an einer Seite mit einer Silikonmembran 12 abgeschlossen ist, welche den eigentlichen Zugang bildet. Sowohl die Pumpeneinheit 3 als auch der Port 5 sind zur Befestigung im subkutanen Gewebe vorgesehen und weisen hierzu entsprechende (vereinfacht dargestellte) Ausnehmungen 13 auf, mit denen die Bauteile im Körper durch Nähen fixiert werden können.

Die Schlauchleitung 10 mündet in dem Reservoir 4. Es handelt sich hierbei um ein flexibles Gefäß, das in den Bauchraum platziert wird. Der dort anstehende hydrostatische Druck von etwa 600 - 800 mm Wassersäule wird über die flexible Außenwand auf die im Reservoir befindliche Flüssigkeit, z. B. eine Kochsalzlösung, weitergeleitet.

Die Pumpeneinheit 3 beinhaltet neben der eigentlichen Pumpe noch ein elektrisch steuerbares Umschaltventil zum Druckausgleich zwischen Reservoir 4 und Blähkörper 2. Weiterhin ist innerhalb der Pumpeneinheit 3 eine Steuer- und Versorgungseinrichtung vorgesehen, die über eine Induktionsschleife bzw. -spule von außen induktiv mit elektrischer Energie bzw. elektrischen Signalen versorgt wird. In der Steuer- und Versorgungseinrichtung wird der induktiv übertragene und dann gepulste Gleichstrom unmittelbar zum Antrieb der Pumpe, also zur Beaufschlagung des piezoelektrischen Antriebs der Pumpe genutzt. Dabei sorgt die Steuerung dafür, dass die Pumpe erst dann mit elektrischer Energie versorgt, das heißt aktiviert wird, wenn ein entsprechendes elektromagnetisches Steuersignal empfangen wird.

Zur Energieversorgung und Steuerung ist ein extrakorporales Versorgungs- und Steuergerät 14 vorgesehen, das eine eigene Stromversorgung in Form eines Akkus beinhaltet. Das Gerät 14 weist eine Kapazitätsanzeige 17 auf, welche die Kapazität des Energiespeichers anzeigt. Über eine Induktionsschleife bzw. -spule entsprechend der in der Pumpeneinheit 3 wird elektrische Energie von dem Gerät 14 induktiv in die Pumpeneinheit 3 übertragen. Zu diesem Zweck ist das Gerät 14 etwa an der Stelle, an der die Pumpeneinheit 3 unter der Haut implantiert ist, an die Körperoberfläche zu führen. Sobald die induktive Stromversorgung der Pumpeneinheit 3 durch das Gerät 14 erfolgt, leuchtet eine Anzeige 15 in Form einer Leuchtdiode und signalisiert dem Anwender, dass nunmehr die Pumpe betätigt werden kann. Hierzu ist am Gerät 14 ein Taster 16 vorgesehen, nach dessen Auslösen die Steuerung des Gerätes 14 ein von der Steuerung innerhalb der Pumpeneinheit 3 empfangenes Signal auslöst, was wiederum die Pumpe für eine vorbestimmte Zeit von beispielsweise 2 Minuten ansteuert. Die Pumpe fördert dann Flüssigkeit aus dem Blähkörper 2 über die Schlauchleitung 6 durch die Pumpe, über die Schlauchleitung 7. das Verteilstück 8 und die Schlauchleitung 10 in das Reservoir 4. Durch Entleeren des Blähkörpers 2 fällt dieser zusammen und gibt in an sich bekannter Weise den Harnröhrendurchgang zur Blasenentleerung frei. Nach der vorbestimmten Zeit wird die Pumpe vom Patienten abgeschaltet. Das Gerät 14 kann dann vom Körper entfernt werden.

Zum Kalibrieren des Systems wird mittel einer Kanüle zunächst durch die Haut und dann durch die Membran 12 des darunterliegenden Ports 5 gestochen, wodurch eine Leitungsverbindung zu dem sonst geschlossenen Fluidsystem geschaffen ist. Es kann nun je nach Erfordernis Flüssigkeit zu oder auch abgeführt werden. Nach Abziehen der Kanüle verschließt die Membran sich und damit das implantierte System selbsttätig. Das implantierte System 1 - 13 wird vollständig montiert und befüllt hergestellt, so dass der operierende Arzt ausschließlich für die bestimmungsgemäße Implantation der Systemteile zu sorgen hat. Die Kalibrierung, das heißt die Anpassung des Drucks an die örtlichen Verhältnisse, kann postoperativ über den Port 5 erfolgen.

### Bezugszeichenliste

- 1 -: Manschette
- 2 -: Blähkörper
- 3 -: Pumpeneinheit
- 4 -: Reservoir
- 5 -: Port
- 6 -: Schlauchleitung
- 7 -: Schlauchleitung
- 8 -: Y-Stück
- 9 -: Schlauchleitung
- 10 -: Schlauchleitung
- 11 -: Metallgehäuse
- 12 -: Silikonmembran
- 13 -: Ausnehmungen
- 14 -: Versorgungs- und Steuergerät
- 15 -: Anzeige
- 16 -: Taster
- 17 -: Kapazitätsanzeige

## Patentansprüche

1. Implantierbare Sphinkterprothese zur Anwendung bei Harninkontinenz, mit einer um die Harnröhre legbaren Manschette (1) mit einem durch Fluiddruck elastisch dilatierbaren und dann die Harnröhre absperrenden Blähkörper (2), mit einem Fluidreservoir (4) und mit einer Pumpe die einerseits mit dem Fluidreservoir (4) und andererseits mit dem Blähkörper (2) leitungsverbunden ist, wobei die Pumpe elektrisch angetrieben und eine extrakorporale Steuer- und Versorgungseinrichtung (14) vorgesehen ist, welche die Pumpe drahtlos mit elektrischer Energie versorgt und steuert, **dadurch gekennzeichnet, dass** zwischen Pumpe und Blähkörper (2) sowie zwischen Pumpe und Reservoir (4) ein elektrisch steuerbares Umschaltventil vorgesehen ist, derart, dass in Abhängigkeit der Schaltstellung der druckseitige Pumpenanschluss mit dem Blähkörper (2) oder dem Reservoir (4) und der saugseitige Pumpenanschluss mit dem Reservoir (4) oder dem Blähkörper (2) leitungsverbunden ist.

2. Sphinkterprothese nach Anspruch 1, **dadurch gekennzeichnet, dass** der Pumpe eine diese steuernde intrakorporale elektronische Steuer- und Versorgungseinrichtung zugeordnet ist, welche drahtlos, vorzugsweise induktiv mit elektrischer Energie versorgt wird und zum Empfang externer Steuerbefehle für die Pumpe ausgebildet ist.

3. Sphinkterprothese nach einem der vorhergehenden Ansprüche, **dadurch gekennzeichnet, dass** die Pumpe eine miniarturisierte Membranpumpe ist deren Antrieb piezoelektrisch erfolgt

4. Sphinkterprothese nach einem der vorhergehenden Ansprüche, **dadurch gekennzeichnet, dass** ein intrakorproraler Port (5) vorgesehen ist, der mit dem Reservoir (4) und/oder dem Blähkörper (2) leitungsverbunden ist.

5. Sphinkterprothese nach einem der vorhergehenden Ansprüche, **dadurch gekennzeichnet, dass** eine Pumpeneinheit (3) vorgesehen ist, welche die Pumpe, die intrakorporale Steuer- und Versorgungseinrichtung und das Umschaltventil umfasst.

6. Sphinkterprothese nach einem der vorhergehenden Ansprüche, **dadurch gekennzeichnet, dass** im Bereich der Manschette (1) ein Drucksensor vorgesehen ist und dass die Pumpe in Abhängigkeit des Drucksensorsignals steuerbar ist.

7. Sphinkterprothese nach einem der vorhergehenden Ansprüche, **dadurch gekennzeichnet, dass** mindestens die Pumpeneinheit (3), der Blähkörper (2), das Reservoir (4), der Port (5) und die zugehörigen Leitungsverbindungen (6-10) als zusammenhängendes, fluidgefülltes und geschlossenes System eine Baueinheit bilden.

8. Sphinkterprothese nach einem der vorhergehenden Ansprüche, **dadurch gekennzeichnet, dass** die extrakorporale Steuer- und Versorgungseinrichtung als mobiles Gerät (14) ausgebildet ist und eine eigene, vorzugsweise wiederaufladbare Energieversorgung aufweist.

9. Sphinkterprothese noch einem der vorhergehenden Ansprüche, **dadurch gekennzeichnet, dass** das mobile Gerät (14) eine optische und/oder akustische Signaleinrichtung (15) aufweist, die ein Signal abgibt, sobald die induktive Kopplung zwischen extrakorporale und intrakorporale Steuer- und Versorgungseinrichtung besteht, wonach Mittel (16) zum Betätigen der Pumpe aktivierbar sind.

## Claims

1. An implantable sphincter prosthesis for the application with urinary incontinence, with a sleeve (1) which may be applied around the urethra, with an inflation body (2) which may be elastically dilated by way of fluid pressure and then blocks the urethra, with a fluid reservoir (4) and with a pump which on the one hand is connected by conduit to the fluid reservoir (4) and on the other hand to the inflation body (2), wherein the pump is electrically driven and an extracorporeal control- and supply device (14) is provided, which in a wireless manner supplies the pump with electrical energy and controls it, **characterised in that** an electrically controllable switch-over valve is provided between the pump and the inflation body (2) as well as between the pump and the reservoir (4), in a manner such that the pressure-side pump connection is connected by conduit to the inflation body (2) or to the reservoir (4), and the suction-side pump connection to the reservoir (4) or to the inflation body (2), in dependence on the switch position.

2. A sphincter prosthesis according to claim 1, **characterised in that** an intracorporeal, electronic control- and supply device which controls the pump, is allocated to this pump, and is supplied with electrical energy in a wireless manner, preferably in an inductive manner, and is designed for receiving external control commands for the pump.

3. A sphincter prosthesis according to one of the preceding claims, **characterised in that** the pump is a miniaturised membrane pump whose drive is effected in a piezoelectric manner.

4. A sphincter prosthesis according to one of the preceding claims, **characterised in that** an intracorporeal port (5) is provided, which is connected by conduit to the reservoir (4) and/or to the inflation body (2).

5. A sphincter prosthesis according to one of the preceding claims, **characterised in that** a pump unit (3) is provided, which includes the pump, the intracorporeal control- and supply device and the switch-over valve.

6. A sphincter prosthesis according to one of the preceding claims, **characterised in that** a pressure sensor is provided in the region of the sleeve (1), and that the pump is controllable in dependence on the pressure sensor signal.

7. A sphincter prosthesis according to one of the preceding claims, **characterised in that** at least the pump unit (3), the inflation body (2), the reservoir (4), the port (5) and the associated conduit connections (6-10) as a coherent, fluid-filled and closed system, form a module.

8. A sphincter prosthesis according to one of the preceding claims, **characterised in that** the extracorporeal control- and supply device is designed as a mobile apparatus (14) and has its own, preferably rechargeable energy supply.

9. A sphincter prosthesis according to one of the preceding claims, **characterised in that** the mobile apparatus (14) comprises an optical and/or acoustic signal device (15) which emits a signal as soon as the inductive coupling between the extracorporeal and intracorporeal control- and supply device exists, whereupon means (16) for actuating the pump may be activated.

## Revendications

1. Prothèse de sphincter implantable pour application en cas d'incontinence urinaire, comportant une manchette (1) pouvant être posée autour de l'urètre, munie d'un corps gonflable (2) dilatable élastiquement par pression hydraulique, puis obturant l'urètre, un réservoir à fluide (4) et une pompe raccordée fonctionnellement d'une part au réservoir à fluide (4) et, d'autre part, au corps gonflable (2), la pompe étant à entraînement électrique et un dispositif extracorporel de commande et d'alimentation (14) étant prévu, lequel alimente et commande la pompe en énergie électrique sans fil, **caractérisée en ce qu'**entre la pompe et le corps gonflable (2) de même qu'entre la pompe et le réservoir (4) est prévue une soupape de commutation pouvant être commandée électriquement de telle façon qu'en fonction de la position de commutation le raccord de pompe côté refoulement est raccordé fonctionnellement au corps gonflable (2) ou au réservoir (4) et le raccord de pompe côté aspiration est raccordé fonctionnellement au réservoir (4) ou au corps gonflable (2).

2. Prothèse de sphincter selon la revendication 1, **caractérisée en ce qu'**à la pompe est associé un dispositif de commande et d'alimentation électronique intracorporel pilotant celle-ci, lequel est alimenté en énergie électrique sans fil, de préférence de façon inductive, et est réalisé pour recevoir des instructions de commande externes pour la pompe.

3. Prothèse de sphincter selon l'une des revendications précédentes, **caractérisée en ce que** la pompe est une pompe à diaphragme miniaturisée à commande piézoélectrique.

4. Prothèse de sphincter selon l'une des revendications précédentes, **caractérisée en ce qu'**est prévu un port intracorporel (5) qui est raccordé fonctionnellement au réservoir (4) et/ou au corps gonflable (2).

5. Prothèse de sphincter selon l'une des revendications précédentes, **caractérisée en ce qu'**est prévue une unité de pompe (3) qui comprend la pompe, le dispositif de commande et d'alimentation intracorporel et la soupape de commutation.

6. Prothèse de sphincter selon l'une des revendications précédentes, **caractérisée en ce qu'**un capteur de pression est prévu dans la zone de la manchette (1) et que la pompe peut être commandée en fonction du signal du capteur de pression.

7. Prothèse de sphincter selon l'une des revendications précédentes, **caractérisée en ce qu'**au moins l'unité de pompe (3), le corps gonflable (2), le réservoir (4), le port (5) et les liaisons fonctionnelles (6 à 10) associées forment, en tant que système intégré, rempli de fluide et fermé, une unité modulaire.

8. Prothèse de sphincter selon l'une des revendications précédentes, **caractérisée en ce que** le dispositif de commande et d'alimentation extracorporel est réalisé en tant qu'unité mobile (14) et offre une alimentation propre en énergie, de préférence rechargeable.

9. Prothèse de sphincter selon l'une des revendications précédentes, **caractérisée en ce que** l'unité mobile (14) présente un dispositif de signalisation optique et/ou acoustique (15) qui émet un signal dès que se produit le couplage inductif entre les dispositifs de commande et d'alimentation, respectivement intracorporel et extracorporel, des moyens (16) d'actionnement de la pompe pouvant alors être activés.
